# EUROPEAN PATENT APPLICATION

(11) **EP 1 064 891 A1**
(43) Date of publication of application: **03.01.2001**
(21) Application number: 00305456.6
(22) Date of filing: 29.06.2000
(51) Int. Cl.: A61F 5/055

(54) **Cervical Collar**

(30) Priority: 02.07.1999 GB 9915379
(71) Applicant: Gilbert & Mellish Limited, West Heath, Birmingham B31 3PH (GB)
(72) Inventor: Cowley, Heather, Baddeley Green, Stoke-on-Trent ST2 7HE (GB)
(74) Representative: Pearce, Anthony Richmond

(57) **Abstract**

A cervical collar includes a flexible support member (12), a retention band (14) and a cord (16). The retention band (14) is fixed to a surface of the support member (12), and the cord (16) passes slidably between the support member (12) and the retention band (14). The cord (16) forms a loop whose size can be adjusted by sliding the cord (16) relative to the support member (12) so that the collar can be passed over the head and the cord (16) tightened to retain the support member (12) in position around the neck of a user.

## Description

This invention relates to a cervical collar. More particularly it relates to a cervical collar for self-application.

The use of cervical collars to limit the movement of a patient's head and neck in cases of whiplash and other spinal trauma is well known. The use of a cord to retain the parts of the collar in position around the patient's neck is also known, see for example UK Patent No. 2 293 537.

However, the current types of collars are not easily fitted, unaided, by the patient as the collar is either made of two parts which must be joined together or require the collar to be manually formed to fit around the patient's neck. Both the joining and filling procedures require the use of two hands and a high degree freedom of movement, this can prove difficult for patients who have suffered neck and other upper body injuries or who are arthritic.

It is an object of the present invention to provide a cervical collar which mitigates the above difficulty.

According to the present invention there is provided a cervical collar including a flexible support member, retention means and a cord or the like, the retention means being fixed to a surface of the member, and the cord passing slidably between the support member and the retention means, and forming a loop whose size can be adjusted by sliding the cord relative to the member.

The loop can be varied in size from a large size, to allow it to be passed over the head of a patient, to a smaller size, in order to cause the support member to flex and fit around the neck of the patient.

Preferably the retention means is of unitary construction. More preferably the retention means is a band. Even more preferably the band extends over substantially the length of the support member.

Desirably there is sufficient friction between the cord and the retention means to hold the cord. Alternatively the ends of the cord pass through a fastening which retains the cord in position, in use. The fastening may be a spring loaded grip, a clip, a toggle or a clamp.

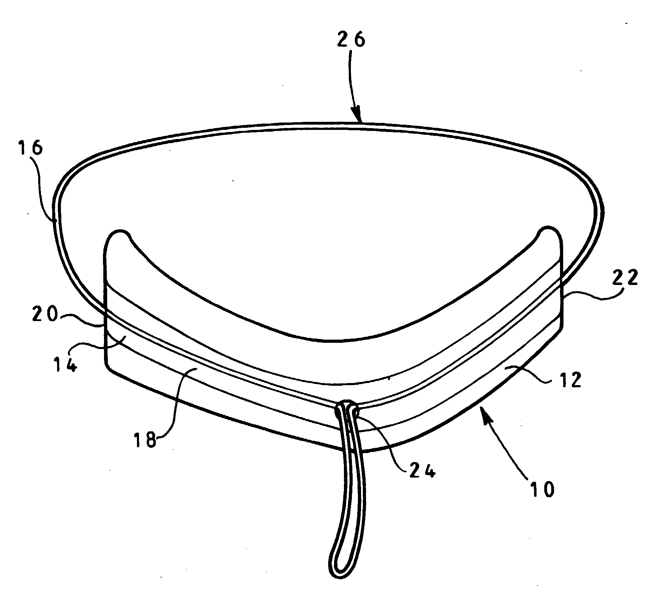

The invention will now be described, by way of example only, with reference to the accompanying drawing which is a schematic perspective view of a cervical collar according to the present invention.

A cervical collar 10 includes a rectangular flexible support member 12, a rectangular band 14 and a cord 16.

The band 14 is fixed to the support member 12, being longitudinally coextensive, and defining a passageway 18 therewith. A pair of openings 20, 22 are defined at each end of the passageway 18. The band 14 has an aperture 24 at its mid-point.

The cord 16 passes into the passageway 18 through the respective openings 20,22 therein and exits the passageway 8 through the aperture 24 so that end regions of the cord 16 project through the aperture 24 thus defining a loop 26, the support member 12 being internal of the loop 26.

In use, the loop 26 is passed over a patient's head and the support member 12 is positioned in contact with the patient's neck, ideally with the aperture 24 in an anterior position. The cord 16 is drawn outwardly from the passageway 18 through the aperture 24 thereby reducing the size of the loop 26. The frictional force between the cord 16 and the surface of the support member 12 prevents the cord 16 from slipping inwardly into the passageway 18. As the size of the loop 26 is reduced the support member 12 is formed into a substantially circular tubular shape around the neck of the patient (not shown), thereby providing support to the patient's neck and head.

In a further embodiment (not shown), the cord passes through a fastening which holds the cord thus preventing slippage of the cord into the passageway and maintaining the support member in its tubular shape. It will be appreciated that any form of fastening may be used such as a clip, toggle or clamp.

## Claims

1. A cervical collar including a flexible support member (12), retention means (14) and a cord or the like (16), the retention means (14) being fixed to a surface of the member (12), and the cord (16) passing slidably between the support member (12) and the retention means (14), and forming a loop whose size can be adjusted by sliding the cord (16) relative to the support member (12).

2. A cervical collar as claimed in claim 1, wherein the retention means (14) is of unitary construction.

3. A cervical collar as claimed in claim 2, wherein the retention means (14) is a band.

4. A cervical collar as claimed in claim 3, wherein the band (14) extends over substantially the length of the support member (12).

5. A cervical collar as claimed in any preceding claim, wherein there is a frictional engagement between the cord (16) and the retention means (14) such as to releasably hold the cord (16) in a set position in use.

6. A cervical collar as claimed in any one of claims 1 to 4, wherein the ends of the cord (16) pass through a fastening which retains the cord (16) in a set position, in use.
